Europäisches Patentamt

European Patent Office

Office européen des brevets

(11) Veröffentlichungsnummer: **0 510 319 A2**

# EUROPÄISCHE PATENTANMELDUNG

(21) Anmeldenummer: **92102644.9**

(22) Anmeldetag: **18.02.92**

(51) Int. Cl.⁵: **C12N 1/00**, C12P 13/08,
//(C12N1/00,C12R1:15,1:13)

(30) Priorität: **25.04.91 DE 4113471**

(43) Veröffentlichungstag der Anmeldung:
**28.10.92 Patentblatt 92/44**

(84) Benannte Vertragsstaaten:
**BE DE DK ES FR GB NL SE**

(71) Anmelder: **Degussa Aktiengesellschaft**
**Weissfrauenstrasse 9**
**W-6000 Frankturt am Main 1(DE)**

(72) Erfinder: **Bachmann, Bernd, Dr.**
**Meyerfeld 10 a**
**W-4806 Werther(DE)**
Erfinder: **Kircher, Manfred, Dr.**
**Liethstück 16 a**
**W-4800 Bielefeld 1(DE)**

(54) **Verfahren zur Erhöhung der Leistungsfähigkeit L-Lysin ausscheidender coryneformer Mikroorganismen.**

㊼ Die Erfindung betrifft ein Verfahren zur Erhöhung der Leistungsfähigkeit von L-Lysin ausscheidenden, AEC-resistenten coryneformen Stämmen von Mikroorganismen, das dadurch gekennzeichnet ist, daß man bei diesen Stämmen eine Oxalysin-Resistenz induziert.

EP 0 510 319 A2

Die Erfindung betrifft ein Verfahren zur Erhöhung der Leistungsfähigkeit L-Lysin ausscheidender coryneformer Mikroorganismen.

Die essentielle Aminosäure L-Lysin ist als Nahrungs- und Tierfutterzusatz, sowie als Wirkstoff und Bestandteil von pharmazeutischen Produkten von großer industrieller Bedeutung.

Für die Herstellung von L-Lysin ist die Fermentation das bedeutendste Verfahren. Vorallem coryneforme Bakterien der Gattungen Corynebacterium und Brevibacterium werden in der Produktion eingesetzt.

Durch Mutationen ist die Regulation der Lysin-Biosynthese dieser Stämme so verändert, daß sie Lysin über den Eigenbedarf hinaus produzieren und in das Medium ausscheiden.

Derartige Überproduzenten erhält man durch Suche nach Mutanten, in denen einzelne Schritte des Aminosäurestoffwechsels blockiert sind (z.B. Hse- oder Thr-Auxotrophe), die gegen ein Analogon von Lysin resistent sind oder weitere Mutationen enthalten. Hochleistungsstämme besitzen im allgemeinen mehrere Auxotrophien, eine Analogon-Resistenz oder eine Kombination dieser Mutationen. Eine zusammenfassende Darstellung der Entwicklung von Lysin-Produzenten geben O. Tosaka und K. Takinami (Progr. Ind. Microbiol.(Biotechnol. Amino Acids) 24 (1986), 152-172).

Die Suche nach Mutanten, die L-Lysin produzieren, wird als Screening bezeichnet. Im Screening werden in einem Ausgangsstamm mittels gebräuchlicher chemischer oder physikalischer Mutagene (z. B. MNNG oder UV) zufällige Mutationen induziert und mit üblichen mikrobiologischen Methoden Mutanten selektioniert. Entscheidend für den Erfolg des Screening ist nun die Auswahl des Selektionsmittels und dessen geeignete Anwendung.

Für die Selektion von Lysinproduzenten wird im allgemeinen ein Strukturanalogon von Lysin eingesetzt. Die das Wachstum hemmende Wirkung dieses Analogons wird durch L-Lysin aufgehoben. Unter Mutanten, die gegen das Analogon resistent sind, findet man deshalb auch solche mit erhöhter L-Lysinproduktion.

Ein bekanntes Beispiel für eine solche Verbindung ist AEG (S-[2-Aminoethyl]-Cystein). AEC unterscheidet sich von L-Lysin nur dadurch, daß in Position 4 das Kohlenstoffatom gegen ein Schwefelatom ausgetauscht ist. Seine lysinanaloge Wirkung ist am Beispiel der Hemmung der Aspartat-Kinase nachgewiesen. Dabei bewirkt AEC allein in vitro keine Hemmung des Enzyms, während die für die natürliche Effektorkombination Lysin/Threonin bekannte konzertierte Hemmung auch für die Kombination AEC/Threonin nachweisbar ist (Tab.1). Dies kann als Ursache für die wuchshemmende Wirkung von AEC gelten, die durch zusätzliche L-Threonin Supplementierung deutlich verstärkt wird.

Für die Selektion von L-Lysin produzierenden Mutanten mittels AEC wird daher üblicherweise die Kombination AEC/Threonin eingesetzt.

In der Literatur als AEC-resistent beschriebene Lysinproduzenten zeigen entsprechend eine zusätzliche Thr-Resistenz. Lysinproduzenten dieses Typs sind aus der Literatur bekannt (H. Kase, K. Nakayama; Acric. Biol. Chem. 38 (1974), 993 bis 1000; S N. Kara-Murza et al.; Prikladnaya Biokhimiya Mikrobiologiya 16 - (1980) 868 bis 875; US. Pat. 3,707,441).

Es wird nun häufig versucht, die Leistungsfähigkeit dieser Mutanten durch Einführung weiterer Mutationen zu steigern. Bekannt ist die Kombination mit Auxotrophien, die sich mit dem Fachmann geläufigen Methoden leicht induzieren lassen (US-Pat. 3,708,395; US-Pat. 3,825,472; J. Plachy, Acta Biotechnol. 9 - (1989) 3,291bis 293; A. Sassi et al.; Biotechnol. Lett. 12 (1990) 4, 295 bis 298).

Weiterhin ist die Kombination mit weiteren Resistenzen bekannt, insbesondere die Resistenz gegen Antibiotika (DE-OS 27 30 964).

Aufgabe der Erfindung ist es, durch weitere Mutationen die Leistungsfähigkeit L-Lysin ausscheidender coryneformer Mikroorganismen zu steigern.

Gegenstand der Erfindung ist ein Verfahren zur Erhöhung der Leistungsfähigkeit von L-Lysin ausscheidenden, AEC-resistenten coryneformen Stämmen von Mikroorganismen, das dadurch gekennzeichnet, daß man bei diesen Stämmen eine Oxalysin-Resistenz induziert.

Dies erfolgt in der Weise, daß der Ausgangsstamm gebräuchlichen chemischen oder physikalischen Mutagenen ausgesetzt wird, z. B.
MNNG: N-Methyl-Nitroso-Guanidin
oder UV.

Die Selektion der geeigneten coryneformen Bakterien, die bevorzugt den Gattungen Corynebacterium, insbesondere Corynebacterium glutamicum, oder Brevibacterium, insbesondere Brevibacterium flavum oder lactofermentum, angehören, erfolgt nach allgemein bekannten mikrobiologischen Methoden.

Bei Oxalysin(O- [2-Aminoethyl]-Serin handelt es sich um ein Strukturanalogon von L-Lysin, das sich von diesem nur dadurch unterscheidet, daß in Position 4 das Kohlenstoffatom gegen ein Sauerstoffatom ausgetauscht ist.

Die Tatsache, daß man die Resistenz gegen ein zweites Lysinanalogon induzieren und danach selektionieren kann, überrascht an sich. Es wirkt im vorliegenden Fall umso erstaunlicher, da für Oxalysin die lysinanaloge Wirkung ebenso wie für AEC durch die Hemmung der Aspartatkinase erfolgt.

Dabei zeigt sich, daß DL-Oxalysin wie L-Lysin in vitro hemmend wirkt, wobei die Wirkung durch L-Threonin verstärkt wird (Tab. 1 und 2).

Im Gegensatz dazu und im Gegensatz zur Wirkung von AEC zeigt sich aber, daß Threonin auf die durch Oxalysin verursachte Wuchshemmung keinen Einfluß nimmt (Tab.3). Das ermöglicht es, Oxalysin-resistente Lysinproduzenten auch ohne zusätzliche Supplementierung mit Threonin zu selektionieren.

Dies hat den großen Vorteil, daß sich Mutanten nicht in durch Threonin beeinflußten Stoffwechselschritten, die nicht zur Lysinproduktion beitragen, anreichern. Außerdem wird durch die alleinige Anwendung des Lysinanalogons die Methodik des Selektionsverfahrens vereinfacht.

AEC-resistente coryneforme Bakterien, die auf diese Weise selektioniert werden, weisen eine gegenüber den Ausgangsstämmen gesteigerte Lysinproduktion auf. Dabei ist es unwesentlich, welche Mutationen (Resistenzen, Auxotrophien) diese Stämme sonst noch tragen.

Beispiele

Die Beispiele beziehen sich auf Mutanten von Corynebacterium glutamicum (ATCC13032), die mit Hilfe chemischer (MNNG) oder physikalischer (UV) Mutagene gewonnen wurden.

Beispiel 1

DM290-2 (hse⁻, AEC$^r$) wird über Nacht in Standard I Bouillon (Merck Art. 7882) angezogen, gegen physiologische Kochsalzlösung (0,9 % Nacl) gewaschen, mit Ethylmethansulfonat (LD$_{37}$) mutagenisiert und auf Gradientenplatten, die Oxalysin enthalten, ausgespatelt. Die Gradientenplatten enthalten das Medium BMCG (Liebl et al., Appl. Microbiol. Biotechnol. (1989) 32:205 bis 210), supplementiert mit 200 ug/ml DL-Homoserin. Die untere Gradientenschicht enthält 2,5 g/l DL-Oxalysin. Nach 5 Tagen Inkubation bei 30° Cwerden resistente Kolonien abgeimpft.

Zur Prüfung der Lysinproduktion werden resistente Kolonien in CASO-Bouillon (Merck Art. 5459) 16 h inkubiert (300rpm, 30° C). Dieses Suspension wird 1:10 in 9 ml Medium MP (240 g/l Melasse, 100 ml/l Sojamehlhydrolysat, 12 g/l Ammoniumsulfat, 10 g/l Calciumcarbonat, pH7) in 100 ml-Erlenmeyer-Kolben mit Schikane verdünnt und 48 h inkubiert (30° C, 300 rpm). Nach 48 h wird die Fermentationsbrühe abzentrifugiert und die Lysinkonzentration im Überstand mittels Aminosäureanalyse bestimmt.

| Stamm | Phänotyp | Lys*Hcl (g/l) |
|---|---|---|
| DM290-2 | hse⁻,AEC$^r$ | 36,5 |
| DM431 | hse⁻,AEC$^r$, Oxa$^r$ | 39,4 |
| DM435 | hse⁻,AEC$^r$, Oxa$^r$ | 42,6 |
| DM436 | hse⁻,AEC$^r$, Oxa$^r$ | 39,8 |
| DM437 | hse⁻,AEC$^r$, Oxa$^r$ | 42,8 |

Beispiel 2

DM282-2 (leu⁻, AEC$^r$) wird über Nacht in Standard I Bouillon (Merck Art. 7882) angezogen, gegen physiologische Kochsalzlösung (0,9 % Nacl) gewaschen, mit Methylnitrosoharnstoff (LD$_{37}$) mutagenisiert und auf Gradientenplatten, die Oxalysin enthalten, ausgespatelt. Die Gradientenplatten enthalten das Medium BMCG, supplementiert mit 100 $\mu$g/ml L-Leucin. Die untere Gradientenschicht enthält 2,5 g/l DL-Oxalysin. Nach 5 Tagen Inkubation bei 30° C werden resistente Kolonien abgeimpft.

Die Prüfung der Lysinproduktion erfolgt wie in Beispiel 1.

| Stamm | Phänotyp | Lys*Hcl (g/l) |
|---|---|---|
| DM282-2 | leu⁻, AEC$^r$ | 36,1 |
| DM346-1 | leu⁻, AEC$^r$·Oxa$^r$ | 40,5 |

Beispiel 3

3

DM 286-1 (hse⁻, leu⁻, Penʳ, AECʳ) wird über Nacht in Standard I Bouillon (Merck Art. 7882) angezogen, gegen physiologische Kochsalzlösung (o,9 % Nacl) gewaschen, mit Methylnitrosoharnstoff (LD₃₇) mutagenisiert und auf Gradientenplatten, die Oxalysin enthalten, ausgespatelt. Die Gradientenplatten enthalten das Medium BMCG, supplementiert mit 100 μg/ml L-Leucin und 160 μg/ml DL-Homoserin. Die untere Gradientenschicht enthält 2,5 g/l DL-Oxalysin. Nach 5 Tagen Inkubation bei 30° C werden resistente Kolonien abgeimpft.

Die Prüfung der Lysinproduktion erfolgt wie in Beispiel 1.

| Stamm | Phänotyp | Lys*HCl (g/l) |
|---|---|---|
| DM286-1 | hseʳ,leu⁻,Penʳ,AECr | 36,1 |
| DM396-1 | hseʳ,leu⁻,Penʳ,AECʳ,Oxaʳ | 40,5 |

Tab. 1

# Hemmung der ASPARTAT-KINASE aus Corynebacterium glutamicum ATCC 13032

| Aspartat-Kinase | Effektoren | | AK-Restaktivität |
|---|---|---|---|
| [nMol/ml•min] | [mM] | [mM] | [%] |
| | L-Lys | L-Thr | |
| 9862 | 0 | 0 | 100 |
| 9293 | 10 | 0 | 94 |
| 7626 | 50 | 0 | 78 |
| 6971 | 100 | 0 | 71 |
| 4298 | 400 | 0 | 44 |
| 1058 | 10 | 1 | 11 |
| 691 | 10 | 10 | 7 |
| 398 | 100 | 1 | 4 |
| 343 | 100 | 10 | 3 |
| | AEC | L-Thr | |
| 9139 | 100 | 0 | 93 |
| 373 | 100 | 10 | 4 |
| 3168 | 10 | 10 | 32 |
| | Oxalys | L-Thr | |
| 5495 | 100 | 0 | 55 |
| 87 | 100 | 10 | 1 |
| 2425 | 10 | 10 | 25 |

Erläuterung:     Die Hemmeffekte wurden in einem zellfreien dialysierten Rohextrakt gemessen.

Die Messung der Aspartat-Kinase ist beschrieben in:

G.Thierbach, J.Kalinowski, B.Bachmann, A.Pühler; Cloning of a DNA fragment from Corynebacterium glutamicum conferring aminoethyl cysteine resistance and feedback resistance to aspartokinase; Appl. Microbiol. Biotechnol. (1990) 32:443-448.

Tab. 2

| Hemmbarkeit der Asp-Kinase aus C.glutamicum ATCC 13032 | | | | | |
|---|---|---|---|---|---|
| D,L-Oxalysin [mM] | L-Thr [mM] | Aspartat-Kinase % Restaktivität | | L-Lys [mM] | L-Thr [mM) |
| 0 | 0 | 100 | 100 | 0 | 0 |
| 1 | 0 | 98 | - | 1 | 0 |
| 10 | 0 | 92 | 95 | 10 | 0 |
| 100 | 0 | 42 | 64 | 100 | 0 |
| 10 | 1 | 49 | 11 | 10 | 1 |
| 10 | 10 | 27 | 5 | 10 | 10 |
| 100 | 10 | 0 | 1 | 100 | 10 |

Tab. 3

| Wuchshemmung von Corynebacterium glutamicum (ATCC13032) durch AEC oder Oxalysin | | | | | |
|---|---|---|---|---|---|
| Kontrolle | AEC 25mM | Thr 25mM | AEC/Thr 25mM/25mM | Oxa 3mM | Oxa/Thr 3mM/3mM |
| + + + | + + | + + + | - | + | + |

-: keine Wuchs; +: Wuchs (Medium BMCG [Liebl et al.; Appl. Microbiol. Biotechnol. 32 (1989), 205-210], supplementiert mit 0,1 M MOPS; 30°C, 48h]

**Patentansprüche**

1. Verfahren zur Erhöhung der Leistungsfähigkeit von L-Lysin ausscheidenden, AEC-resistenten coryneformen Stämmen von Mikroorganismen, dadurch gekennzeichnet, daß man bei diesen Stämmen eine Oxalysin-Resistenz induziert.

2. Verfahren gemäß Anspruch 1,
dadurch gekennzeichnet, daß man die Selektion der mutierten Stämme in Gegenwart von DL-Oxalysin ohne L-Threonin-Supplementierung vornimmt.

3. Verfahren gemäß den Ansprüchen 1 oder 2,
dadurch gekennzeichnet, daß man Corynebacterium glutamicum einsetzt.

4. Verfahren gemäß den Ansprüchen 1 oder 2,
dadurch gekennzeichnet, daß man Brevivacterium flavum oder lactofermentum einsetzt.

5. Verfahren gemäß mindestens einem der Ansprüche 1 bis 4,
dadurch gekennzeichnet, daß die verwendeten Stämme weitere Resistenzen oder Auxotrophien aufweisen.